(19) Europäisches Patentamt
European Patent Office
Office européen des brevets

(11) **EP 3 145 708 B1**

(12) **EUROPÄISCHE PATENTSCHRIFT**

(45) Veröffentlichungstag und Bekanntmachung des Hinweises auf die Patenterteilung:
**25.03.2020   Patentblatt 2020/13**

(21) Anmeldenummer: **15729086.7**

(22) Anmeldetag: **19.05.2015**

(51) Int Cl.:
*B32B 5/00* (2006.01)        *B32B 5/02* (2006.01)
*B32B 7/00* (2019.01)        *B32B 7/04* (2019.01)
*B32B 7/12* (2006.01)        *B32B 9/00* (2006.01)
*B32B 15/00* (2006.01)       *B32B 15/08* (2006.01)
*B32B 17/00* (2006.01)       *B32B 19/00* (2006.01)
*B32B 19/04* (2006.01)       *B32B 23/00* (2006.01)
*B32B 27/00* (2006.01)       *B32B 27/06* (2006.01)

(86) Internationale Anmeldenummer:
**PCT/EP2015/061022**

(87) Internationale Veröffentlichungsnummer:
**WO 2015/177168 (26.11.2015 Gazette 2015/47)**

(54) **FLEXIBLE PCM-FLÄCHENGEBILDE**

FLEXIBLE PCM SHEET MATERIALS

STRUCTURE PLANE FLEXIBLE EN MATÉRIAU À CHANGEMENT DE PHASE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **19.05.2014   DE 102014007219**

(43) Veröffentlichungstag der Anmeldung:
**29.03.2017   Patentblatt 2017/13**

(73) Patentinhaber: **Smartpolymer GmbH**
**07407 Rudolstadt (DE)**

(72) Erfinder:
• **BÜTTNER, Dirk**
  **12207 Berlin (DE)**
• **SCHÜTZ, Angelo**
  **07407 Rudolstadt (DE)**
• **GEISSENHÖNER, Martin**
  **07407 Rudolstadt (DE)**

(74) Vertreter: **Plate, Jürgen**
**Plate Schweitzer Zounek**
**Patentanwälte**
**Rheingaustrasse 196**
**65203 Wiesbaden (DE)**

(56) Entgegenhaltungen:
DE-A1-102012 218 378        US-A1- 2011 193 008
US-A1- 2012 064 327        US-B1- 6 319 599

**Beschreibung**

Die Erfindung betrifft flexible PCM-Flächengebilde mit hoher latenter Wärmeenergie-Speicherdichte zum Zwecke des Wärmemanagements. Das flexible PCM-Flächengebilde wird gebildet aus einer flexiblen Trägerstruktur und darauf separat in spezieller Geometrie angeordneten Phasenwechselmaterialkörpern. Die Phasenwechselmaterialkörper selbst umfassen geometrisch definierte Strukturen aus polymergebundenem Phasenwechselmaterial. Die flexiblen PCM-Flächengebilde zeichnen sich durch eine hohe latente Wärmespeicherkapazität und optimierte Wärmeleitfähigkeiten aus, sind auch bei Temperaturänderungen und nach Phasenübergängen formstabil und können problemlos gerollt, gefaltet, gewickelt oder zugeschnitten werden und sowohl ein- oder mehrlagig transportiert, gelagert, verarbeitet oder verwendet werden.

Aus US20020164474 sind flexible Wärmeregulierungsmaterialien bekannt, die Verwendung finden in Socken, als Schuhinnenfutter oder anderen Bekleidungsstücken. Um ein Ausbluten der Phasenwechselmaterialien (PCM) in der flüssigen Phase zu verhindern, werden diese PCM verkapselt und in einem flexiblen Matrixmaterial verteilt. Ein anderer Grund für die Verkapselung ist die Verhinderung der Bildung von unflexiblen starren PCM-Aggregaten bei der Abkühlung. Wenn nicht mit Verkapselung gearbeitet wird, werden die PCM fest absorbiert in Absorbern oder Superabsorbern, wie Polyacryl oder Carboxylmethylcellulose. Das Matrixmaterial ist vorzugsweise ein flexibles Polymer oder ein Polymerschaumstoff. Weitere Ausführungsformen betreffen Laminate mit Isolierschichten außen und einer PCM enthaltenden Schicht in der Mitte oder eine integrierte Struktur, bei der zuerst eine Polymerschicht gegossen und teilweise vulkanisiert wird, dann eine 2.

Schicht, angereichert mit PCM gegossen wird und ebenfalls teilvulkanisiert wird und danach eine 3. Schicht Matrixmaterial darauf gegossen wird und der Verbund vollständig vulkanisiert wird. Der Nachteil dieser Lösung besteht darin, dass bei flüssig-fest PCM eine Verkapselung notwendig ist, die Kapseln nicht beliebige Formen annehmen können, bzw. beliebig verformbar sind und der Gesamtverbund in seiner Flexibilität eingeschränkt ist. GB2495938 beschreibt ein Energiespeichersystem, bei dem eine Vielzahl von Kapseln mit PCM Material auf einem oder zum Teil in einem Träger fixiert ist. Der Träger kann starr (Platte) oder flexibel (Blatt) sein. Die Kapseln werden aus polymerem (PVC) oder metallischem Material (Aluminium) gebildet. Zur Verbesserung der Wärmeleitfähigkeit wird das PCM Material mit Graphit oder Metallpulvern gemischt. Die Kapseln können das gleiche PCM-Material oder verschiedene PCM-Materialien enthalten. Entweder werden die Kapseln auf dem Träger fixiert oder der Träger besitzt Aufnahmestellen (Mulden) für das PCM, das PCM wird in die Aufnahmestellen gefüllt und abschließend werden die gefüllten Aufnahmestellen versiegelt. Nachteil ist auch hier, dass das PCM-Material eingekapselt werden muss und die Flexibilität eingeschränkt ist. Zudem ist, wie bei US20020164474, der Strömungswiderstand beim Beladen und Entladen von Wärmemengen mit flüssigen oder gasförmigen Wärmeträgermedien sehr hoch und dadurch die Wärmeübertragung behindert. Aufgrund der beschriebenen notwendigen Verkapselung geht viel Masse und Volumen für das PCM verloren, so dass die Gesamtkapazität stark reduziert wird und so kaum noch nennenswerte Kapazitäten im Gesamtsystem vorhanden sind. Sollte die Verkapselung eine Leckage erleiden, kommt das verflüssigte PCM mit seiner Umgebung in Kontakt. Entsprechende Flächengebilde können auch nicht einfach durch Zuschneiden in die richtige Form gebracht werden. Die Havariehäufigkeit ist eine statistische Größe die nicht vorhergesagt werden kann. So kann es bei einer zu dünnen Wandstärke zum Totalausfall des Systems kommen. Sollte anderseits die Umhüllungsschichtdicke zu stark gewählt werden, tritt das o. g. Szenario der Enthalpie-Reduzierung ein. Bei der Patentanmeldung US2002164474 besteht die grundlegende Funktion des PCM in der Wärmeleitung. Durch die Einbringung in beschriebene offene und/oder geschlossenzellige Schäume werden thermisch sehr träge Systeme geschaffen, Konvektion bringt hier aufgrund der sehr geringen Oberfläche und schlechten Wärmeleitung keine Verbesserung. Sollte ein makroverkapseltes PCM (z. B. kunststoffumhüllt) auf einer flexiblen Matrix aufgeklebt werden, bestimmt immer die (feste) Größe dieses Elements den Biege- und Wickelradius sowie die Konfektionierbarkeit. Eine Makroverkapselung darf nicht geschnitten werden, da der Inhalt austritt. Das betrifft ebenso Beschädigungen im Einsatz (z. B. Einbringung in eine Wand). DE10022287 beschreibt ein 3D-Abstandsgewirke mit zwischen den Lagen angeordneten Teilchen eines Latentwärmespeichermaterials. Beschrieben ist ein Hineindrücken unverkapselter PCM-Teilchen in Öffnungen einer Schicht des Abstandsgewirkes/-gewebes. Damit diese PCM-Teilchen während des Gebrauchs oder beim Waschen nicht herausfallen, wird die PCM-Teilchen enthaltende Schicht mit einer Abdeckung mit kleineren Öffnungen als die Größe der PCM-Teilchen oder mit einer Folie belegt. Da die PCM-Teilchen nicht fest verbunden sind mit dem Abstandsmaterial, kann dieses nicht beliebig zugeschnitten werden. Da das Abstandsmaterial als Trägermaterial mehrere Millimeter Höhe hat, ist es nicht beliebig flexibel. Das entgegengehaltene Abstandsgewirke ist nur gut rollbar, wenn es auf der einen Seite noch offenzellig ist. Werden die offenen Kammern nach dem Befüllen mit PCM-Teilchen versiegelt (flächig verklebt) lässt es sich nicht mehr zusammenrollen. Der Wickelkern im Vergleich zum Durchmesser eines PCM-Teilchens ist bei Abstandsgewirken sehr viel größer. Zudem ist die Beladungsdichte mit den PCM-Teilchen durch die Anzahl der Öffnungen eingeschränkt und die Anströmbarkeit der PCM-Teilchen durch flüssige oder gasförmige Medien ist sehr begrenzt.

In der US 2012/0064327 A1 ist ein PCM-Compound beschrieben, das zu 60 bis 10 Gew.-% aus einem Trägermaterial und zu 40 bis 90 Gew.-% aus einem Phasenwechselmaterial besteht. Es kann zu Fasern oder Folien extrudiert werden.

Die Aufgabe besteht darin, einen vollkommen flexiblen wärmeregulierenden Verbundstoff zu schaffen, der ohne Verkapselung des PCM auskommt, auch bei Temperaturänderungen und Phasenübergängen formstabil bleibt und eine optimierte Wärmeübertragungsfähigkeit durch die besondere geometrisch variable Anordnung der PCM-Formkörper auf dem Flächengebilde besitzt. Dieser Verbundstoff soll zudem zuschneidbar, rollbar und stapelbar sein und zur Verbesserung der Wärmeübertragung soll die Durchströmbarkeit für flüssige und gasförmige Medien einstellbar sein.

Die Aufgabe wird dadurch gelöst, dass eine plastifizierte Masse eines polymergebundenen Phasenwechselmaterials in geometrisch definierten Strukturen auf einer Trägerstruktur fest fixiert wird. Die Fixierung erfolgt dabei in der Art und Weise, dass das schmelzverflüssigte polymergebundene Phasenwechselmaterial (im Folgenden mit PCM abgekürzt) auf die Trägerstruktur als sphärische, quadratische, rechtwinklige oder polygonale Formkörper mit einer Dicke von 1 bis 10 mm, bevorzugt 1 bis 5 mm, durch Spritzguss, Aufspritzen, Aufstreichen oder druckloses Vergießen mittels einer Formvorrichtung kontinuierlich oder diskontinuierlich aufgebracht wird. Diese sphärischen oder polygonalen Formkörper aus polymergebundenem Phasenwechselmaterial (PCM) werden im folgenden PCM-Polymerangussstücke genannt. Die Trägerstrukturen sind im Wesentlichen zweidimensional, d.h. die Höhe dieser Strukturen ist im Vergleich zu deren Länge und Breite vernachlässigbar. Als vorteilhafte Trägerstrukturen erwiesen sich Gewebe, besonders bevorzugt solche mit einer großen Maschenweite, aus Polyamid, Polyester, Polypropylen, Kohlenstoff-, Metall- oder Glasfasern aber auch Naturfasern (Baumwolle, Rayon-Viskosefasern) und Fasermischungen aus diesen genannten Fasern. Weitere mögliche Trägerstrukturen aus diesen Materialien sind textile Strukturen wie Vliesstoffe, Gewirke, Tüll, Gestricke, Geflechte, netzartige Gewebe aus Fasern oder Garnen oder Folienbändchen. Auch Folien oder Membranen sind möglich, vorzugsweise poröse, gelochte oder geflochtene Strukturen. Die Trägerstruktur erfüllt mechanische Aufgaben wie die Zugfestigkeit des Flächengebildes zu verbessern und bestimmt als ein Faktor den Grad der Flexibilität und der Durchströmbarkeit. Sie kann als thermischer oder elektrischer Leiter, z.B. als Widerstandsheizung) plastisch verformbare Matrix oder auch völlig flexibel ausgeführt werden. Verwendet man elektrische Leiter oder Halbleiter, dann kann zusätzlich zum Wärmespeichereffekt der PCM-Materialien auch der Peltier-Effekt ausgenutzt werden. Die separate Anordnung der polymergebundenen PCM-Polymerangussstücke, sowie die Dehnbarkeit und Anpassungsfähigkeit der Trägerstruktur, insbesondere bei einer großmaschigen Struktur, bewirken eine Formstabilität des flexiblen PCM-Flächengebildes selbst bei Phasenwechsel des PCM. Die PCM-Polymerangussstücke sind fest mit der Trägerstruktur verbunden, verklebt oder verschmolzen, sie sitzen oberflächlich oder dringen teilweise in die Trägerstruktur ein.

Gegenstand der Erfindung ist somit ein flexibles PCM-Flächengebilde mit hoher latenter Wärmeenergie-Speicherdichte, gekennzeichnet durch eine flexiblen 2-dimensionale Trägerstruktur mit darauf an der Oberfläche applizierten und mit der Trägerstruktur fest verbundenen, separat in einem Abstand von mindestens 0,5 mm angeordneten geometrisch definierten Strukturen eines polymergebundenen Phasenwechselmaterials, wobei das Phasenwechselmaterial durch mindestens zwei Polymere gebunden ist, von denen mindestens ein Polymer ausgewählt ist aus der Gruppe der styrolhaltigen Blockcopolymere und mindestens ein Polymer ausgewählt ist aus der Gruppe der styrolfreien Ethylen/Butylen-Copolymere, wobei das Flächengebilde auch bei Phasenwechsel formstabil ist, eine latente Wärmeenergie-Speicherdichte von 100 bis 250 J/g bzw. 300 bis 1000 kJ/m$^2$ aufweist und gerollt, gefaltet, gewickelt, zugeschnitten oder mehrlagig konfektioniert werden kann.

Der Werkstoff und die Konstruktion der Trägerstruktur bestimmen, ob das PCM-Flächengebilde eine offene Struktur hat, mit dem sich das PCM verkleben kann oder zusätzlich durch Umschließen der Fasern den Halt bekommt. Die Durchströmbarkeit erleichtert auch das Aufbringen auf andere Formkörper z.B. durch Gießharz oder Kleber. Die Größe der PCM-Körper bestimmt die Flexibilität mit, für den Wärmetransport/-speicherung sind hauptsächlich das PCM-Material und die PCM-Schichtdicke entscheidend. Der Wärmetransport kann auch beeinflusst werden durch die Wahl des Materials für die Trägerstruktur, indem man zum Beispiel wärmeleitende Materialien einsetzt, oder auch durch die Einstellung der Durchströmbarkeit des Flächengebildes. Die Durchströmbarkeit mit flüssigen und gasförmigen Wärmeübertragungsmedien (Wasser, Kühlsole, Luft etc.) durch diese PCM-Flächengebilde und seiner davon hergestellten Produkte (Stapelform, Rollenform etc.) kann im Sinne der Erfindung genau durch die Besatzdichte an PCM-Formkörpern, den seitlichen Abstand zwischen den fixierten PCM-Formkörpern, den Abstand zwischen mehreren PCM-Flächengebilde-Lagen sowie die geometrischen Struktur der Formkörper selbst gezielt eingestellt werden. Durch die so erhaltenen flexiblen PCM-Flächengebilde wird ein PCM-Produkt erlangt, dass einerseits bei thermischer Beanspruchung im Phasenwechseltemperaturbereich seine Formstabilität behält, andererseits für die verschiedensten konstruktiven Anwendungsgebiete von PCM-Materialien gefaltet, gerollt, gewickelt oder zugeschnitten werden kann und durch seine geometrische Trägerstruktur mit darauf separat angeordneten PCM-Polymerangussstücken vor allem ein ungehindertes Durchströmen von Luft, Wasser bzw. anderen Wärmeträgermedien beim Beladen und Entladen der gespeicherten Wärmeenthalpien des PCM-Flächengebildes gewährleisten kann. Die Faltbarkeit oder Rollbarkeit der PCM-Flächengebildelagen wird im Sinne der Erfindung dadurch ermöglicht, dass die PCM-Formkörper auf dem Trägerflächengebilde genau geometrisch, bevorzugt in linearer Reihe, mit geringem Kantenabstand fest fixiert werden. Bevorzugt wird das PCM-Flächengebilde, welches im Sinne der Erfindung bevorzugt als Endlos PCM-Formkörper-Flächengebilde hergestellt wird, entlang der Zwischenabstände dieser PCM-Formkörperreihen in entsprechende Konfektionsgrößen geschnitten. Jedoch führt auch ein Zerschneiden der PCM-Polymerangussstücke nicht zu einem Ausbluten des PCM-Materials und damit auch zu keiner Beeinträchtigung in der Nutzung.

Je geringer der Abstand der PCM-Polymerangussstücke zueinander wird, desto größer wird die Wärmekapazität. Die Abstände sollten nicht unter 0,5 mm sein, da es sonst bei der Lagerung, speziell wenn dabei die Phasenwechseltemperatur überschritten wird, zu flächigen Verklebungen der PCM-Polymerangussstücke miteinander kommen kann. Ein Abstand von 2 mm hat sich in der Herstellung und Nutzung als optimal erwiesen.

Bevorzugt sollte die Dicke (Höhe) 5 mm nicht überschreiten, besonders bevorzugt beträgt diese Dicke 5 mm, soweit es die Anwendung nicht anders erfordert. Bis 5 mm kann das PCM thermisch nahezu voll genutzt werden, bei mehr als 5 mm Höhe nimmt es aufgrund von Oberflächenkühlung durch die Seiten und schlechterer Wärmeleitung nicht mehr am energetischen Austauschprozess teil.

Die plastifizierte Masse des polymergebundenen Phasenwechselmaterials umfasst mindestens 2 Trägerpolymere, ausgewählt aus der Gruppe styrolhaltiger Blockcopolymere, bevorzugt Styrol-Ethylen-Butadien-Styrol - Blockcoplymere und/oder Styrol-Ethylen-Propylen-Blockcopolymere (SEBS bzw. SEEPS), sowie eine styrolfreie Komponente, ausgewählt aus der Gruppe der Polyolefin-Copolymere mit statistischer Verteilung, bevorzugt eine Ethylen-Butylen-Copolymer-Komponente. Die Polyolefin-Copolymer-Komponente weist einen hohen Kristallinitätsgrad auf, bevorzugt im Bereich von 15 bis 30 %. Der Ethylengehalt liegt bevorzugt bei etwa 35 bis 45 Gew.-%, besonders bevorzugt bei etwa 40 Gew.-%. Sie besitzt einen niedrigen Schmelzpunkt (bevorzugt etwa 70 bis 90 °C, besonders bevorzugt 75 bis 85 °C, jeweils bestimmt durch DSC bei 10 K/min Heizrate) sowie niedrige Viskositätswerte (der MFI bei 230 °C und einer Belastung von 2,16 kg beträgt vorzugsweise etwa 0,5 bis 5,0 g/10 min; bestimmt gemäß DIN ISO 1133). Die Molmasse liegt bevorzugt im Bereich von 250.000 bis 500.000 g/mol. Dies dient auch dazu, die Verarbeitungstemperatur für die Angussmasse möglichst niedrig zu halten, damit ein Ausdampfen des PCM-Materials während des Aufbringens auf die Trägerstruktur verhindert werden kann. Die Verarbeitungstemperatur für die Angussmasse liegt in einem Bereich von 100 - 140 °C, bevorzugt bei ca. 120 °C. Mit Hydroxylgruppen terminierte styrolhaltige-Blockcopolymere werden insbesondere verwendet, wenn es sich um polare PCM-Materialien handelt, die selbst Hydroxyl- bzw. Carboxylgruppen aufweisen.

Der Anteil der Styrol-Blöcke in den styrolhaltigen Blockcopolymeren beträgt zweckmäßig etwa 25 bis 35 Gew.-%, bevorzugt etwa 30 Gew.-%. Durch die Verwendung eines mit Hydroxylgruppen terminierten styrolhaltigen-Blockcopolymeren in einem Anteil von 3 bis 35 Gew.% an den Styrol-Blockcopolymeren wird eine PCM-Polymermischung erlangt, die ein Auslaufen bzw. Ausschwitzen der PCM-Komponenten, wie natürliche und synthetische Paraffine, niedrig schmelzende Alkane, Fettalkohole, Fettsäuren, langkettige Dialkylether, Polyethylenglykole, hochkristalline PE-Wachse verhindert, die Anbindung der PCM-Polymermischung an die zumeist polare Trägerstruktur verbessert und gleichzeitig die homogene Einarbeitung von speziellen anorganischen Additiven wie Metalle oder Metalloxide, bevorzugt Zinkoxid, oder wie Graphit, Ruß, Multiwall-Carbon-Nanotubes und auch von organischen Additiven in die PCM-Polymermischung begünstigt. Diese Additive werden eingesetzt um die Wärmeleitfähigkeit und damit den Wärmeaustausch zu verbessern. Des Weiteren lässt sich mit Metalloxiden, reinen Metallpulvern, keramischen Stoffen usw., die eine wesentlich höhere Dichte als 1 g/cm$^3$ haben, die Dichte des PCM einstellen. Das ist für Anwendungen notwendig, bei denen das PCM (Dichte ca. 0,9) in flüssigen Medien (z.B. Wasser) nicht aufschwimmen darf. Weitere Additive können thermochrome Farbstoffe (bevorzugt in einem Anteil von 0,1 bis 3 Gew.-%) sein, die im Bereich der Phasenwechseltemperatur arbeiten und den Fortschritt bzw. Homogenität des Phasenwechselprozesses anzeigen. Weitere Zusätze können Flammschutzmittel im PCM aber auch/und in der Matrix sein.

Die polaren endständigen OH-Gruppen der styrolhaltigen Blockcopolymere bewirken auch eine verbesserte Homogenisierung von Additiven, insbesondere von Zinkoxid, Ruß, Graphit oder Carbon-Nanotubes in den anderen Trägerpolymeren der Trägerpolymerzusammensetzung.

Durch die Verwendung bereits geringer Mengen von 3 bis 8 Gew.-% an Polyolefin-Copolymeren in der Trägerpolymermatrix-Mischung wird einerseits eine viel tiefere Verarbeitungstemperatur sowohl bei der Herstellung der PCM-Polymercompounds möglich, wie auch bei der eigentlichen erfindungsgemäßen Aufbringung der PCM-Polymerangüsse auf die textilen Trägervliese möglich. Andererseits bewirkt diese Copolymere eine größere Volumenausdehnung der Gesamt-Polymermatrix bei der Phasenwechseltemperatur des PCM, die bei rein styrolhaltigen Triblockcopolymeren geringer ist. Eine größere Volumenausdehnung der Polymermatrix bei der Phasenwechseltemperatur bewirkt ein geringeres Ausschwitzen an verflüssigtem Phasenwechselmaterial.

Das Verhältnis styrolhaltiger Blockcopolymere zu den Polyolefin-Copolymeren kann in einem Bereich von 10:1 bis 1:1 variieren, bevorzugt liegt es bei 4:1 bis 2:1.

Zur weiteren Verbesserung des Ausschwitzverhaltens des Phasenwechselmaterials und zur Erlangung einer besseren Oberflächenhaptik können die PCM-Polymerangussstücke mit einer dünnen, dehnbaren Schicht in Form eines Films oder Folie oder einer textilen Fläche überzogen werden. Es kann sich dabei um eine Polymer-, Metall- oder Keramikschicht handeln. Die Schichtdicke des Überzugs beträgt 3 bis 10 μm. Besonders bevorzugt handelt es sich um eine Folienschicht aus Ultramid-1C-Polyamid. Eine dazu vorangehende Oberflächenätzung des butadienischen Anteils des Trägerpolymeren mit einer alkalischen Permanganatlösung bewirkt eine sehr gute Haftung des ULtramid-1C-Films auf den PCM-Polymerangussstücken.

Des Weiteren betrifft die Erfindung solche flexiblen PCM-Flächengebilde, die gefaltet und gerollt werden können, um geometrische Volumenkörper oder mehrlagige textile PCM-Flächengebilde zu erhalten, durch die Luft, Wasser oder andere flüssige Wärmeträgermedien leicht die PCM-PolymerEinheiten umströmen können, so dass die Ein- und Auskopplung von thermischer Energie sehr schnell geschieht. Diese flexiblen PCM-Flächengebilde bestehen aus 1 bis 10 Gew.% an Trägerstrukturen und 90 bis 99 Gew.% des polymergebundenen Phasenwechselmaterials. Das polymergebundene Phasenwechselmaterial umfasst 10 bis 30 Gew.% an Trägerpolymeren und 70 bis 90 Gew.% an PCM. Zusätzlich kann das polymergebundene Phasenwechselmaterial 5 bis 25 Gew.% an anorganischen bzw. organischen Additiven enthalten, bezogen auf das Gewicht des gesamten Angussmaterials bestehend aus PCM, Polymeren und Additiven.

Bei der Phasenübergangstemperatur des Phasenwechselmaterials weisen die erfindungsgemäßen flexiblen PCM-Flächengebilde eine Wärmespeicherenthalpie von bis *zu* 250 J/g auf und ein Flächengewicht von 1 bis 4 kg/m$^2$. Das Flächengewicht bestimmt sich durch die Zwischenräume der PCM-Polymer-Angussstellen auf der Trägerstruktur, durch die Höhe der Polymerangusskörper, der Art und Menge der verwendeten Additive sowie durch das reine Flächengewicht der textilen Trägerstruktur selbst. Die Wärmespeicherenthalpie ist stark abhängig von der Art des verwendeten PCM, vom Anteil des PCM im polymergebundenen PCM, aber auch von der Trägerstruktur (Material und Gewicht) und von der Flächenmasse des polymergebundenen PCM Materials und der Dicke (Höhe) der PCM-Polymerangussstücke. Die Wärmespeicherenthalpie je Flächeneinheit des PCM-Flächengebildes kann bis zu 1000 kJ/m$^2$ betragen.

Der vorteilhafte Effekt der Erfindung liegt darin, dass das PCM-Flächengebilde in seiner dargestellten Form dauerelastisch und formbar bleibt. Über die Anordnung, Teilchendicke und Formgebung der mit dem Matrixmaterial fest verbundenen PCM-Elemente lassen sich Wickelradius und thermische Speicherkapazität bestimmen.

Diese mit PCM-Polymer versehenen flexiblen Flächengebilde eignen sich zur Speicherung und Regulierung von Wärme und Kälte, insbesondere im Gebäudemanagement (Kühlpaneele), Heat-sink-Applikationen in der Elektrotechnik, Wärme-/Kältemanagement im Fahrzeugbau, zur ein- oder mehrlagigen Umhüllung von Rohrleitungen, als gewickelte bzw. in planen Lagen aufeinandergelegte luft- oder wasserdurchströmte Einbauten in Rohren, Kästen etc. Zu den Anwendungen gehört zum Beispiel das Temperieren von Luft. Gestapelte oder gerollte (für Rohre) Flächengebilde würden entsprechende Behälter ausfüllen und als Wärmetauscher fungieren. Weitere Anwendungsmöglichkeiten bestehen in der Temperierung von größeren Flächen an z. B. Maschinen. Dort könnten die Gebilde einfach und um jeden Winkel, Krümmung etc. angebracht werden. Durch das Material und die Konstruktion der Trägerstruktur lassen sich zusätzliche Funktionen einbringen.

Neben mechanischen Stabilisierungen können bei Einsatz metallischer Trägerstrukturen diese "Wärme" oder "Kälte" als elektrische Widerstandsheizung und/oder durch den Peltier-Effekt in das PCM einspeichern.

Insbesondere durch die Dimensionsstabilität der textilen Trägerstruktur im Temperaturbereich des Phasenwechsels des auf der Trägerstruktur applizierten PCM-Polymergemisches können die sonst üblichen Nachteile von reinen PCM-Polymerfolien und PCM-Polymerplatten ausgeglichen werden, wo es stets zu einer unerwünschten Schrumpfung und Verformung im Phasenwechselbereich kam.

Zur Veranschaulichung der Erfindung sind 4 Zeichnungen angefügt.

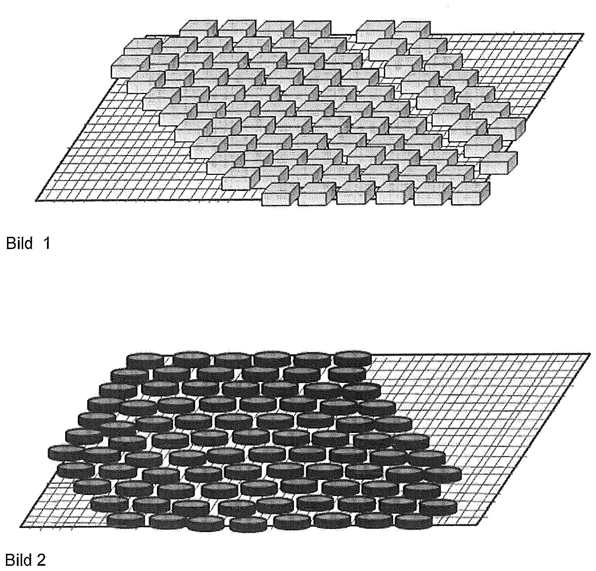

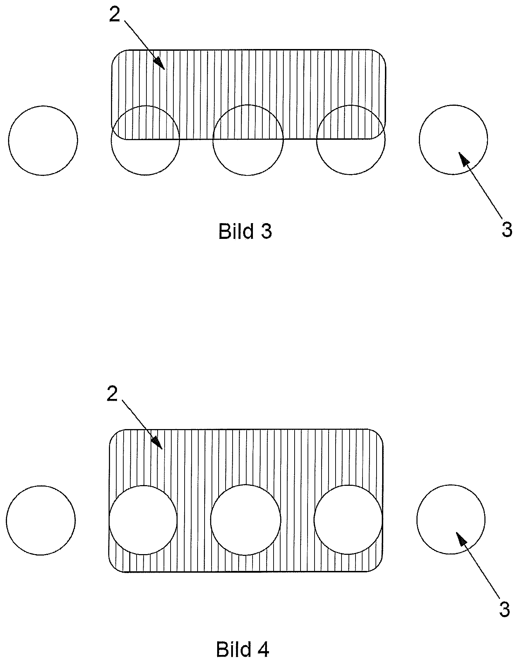

Bild 1 zeigt schematisch eine netzartige Trägerstruktur (1) mit quaderförmigen PCM-Polymer-Angüssen (2) in parallelen Reihen mit konstantem, definiertem Kantenabstand. Es ist deutlich zu erkennen, dass aufgrund der separaten Anordnung der PCM-Polymerangussstücke und der Konstruktion der Trägerstruktur eine gute Durchströmbarkeit für Flüssigkeiten und Gase immer noch gegeben ist. In Bild 2 ist die Form der PCM-Polymerangussstücke (2) zylindrisch, wobei die zylindrischen PCM-Angussstücke wieder in parallelen Reihen auf die Trägerstruktur aufgebracht sind. Bild 3 zeigt eine schematische Darstellung eines Querschnitts der Trägerstruktur (3) mit aufgegossenen PCM-PolymerAngussstücken (2) in geklebter Variante (z.B. Filamente aus Natur-/Synthetikfasern, offenporige Strukturen). Das Verkleben bezieht sich auf die Eigenschaft des polymergebundenen PCM-Materials beim flüssigen Schmelzauftrag mit den Faseroberflächen einen Stoffschluss zu erzeugen, sich also mit der Matrix auf der Oberfläche bis hin zu einer völligen oder teilweisen Durchdringung in Abhängigkeit vom Material der Trägerstruktur zu verkleben. Bild 4 zeigt die schematische Darstellung einer Variante, wo die aufgegossenen PCM-Polymerangussstücke (2) die Fasern (3), die die Trägerstruktur bilden, umschließen (formschlüssige Verbindung). Die plastifizierte Masse des polymergebundenen Phasenwechselmaterials kann in die Zwischenräume der Trägerstruktur eindringen.

**Beispiel 1:**

Mittels eines Doppelschneckenextruders des Typs ZSE 40 (Firma Leistritz) mit einem l/d-Verhältnis von 52 : 1 wird zunächst ein PCM-Polymergranulat aus den Ausgangsstoffen:

- 80 Gew.% PCM-Material (Nacol®ether 12 der Sasol GmbH, langkettiger Dialkylether mit einem Smp. von 32 °C)
- 10 Gew.% Styrol-Blockcopolymer SEEPS (Polystyrol-b-poly(ethylen-ethylen/propylen)-b-polystyrol; ®Septon 4055 der Kuraray Co. Ltd.)
- 5 Gew.% OH-terminiertes Styrol-Block-Copolymer (Septon®HG 252 der Kuraray Co. Ltd.)
- 5 Gew.% kristallines Ethylen-Butylen Copolymer (Typ 6201 B der JSR Dynaron)
hergestellt.

Die Extrusionsdüse war über eine Adapterplatte mit dem Schneidkopf eines Unterwassergranulators (Gala underwater pelletizer, Gala Inc.) verbunden. Es wurden Granulate mit einem Durchmesser von 4 bis 5 mm erlangt.

Die Wärmespeicherkapazität der erhaltenen Granulate bei der Phasenwechseltemperatur des PCM mit einer Schalttemperatur von 32 °C betrug 215 J/g.

Die PCM-Polymer-Granulate wurden in einem Umlufttrockenschrank bei Raumtemperatur von 25 °C getrocknet. Dann wurden diese Granulate in einem senkrecht angeordneten Einschneckenextruder bei 120 °C aufgeschmolzen und die PCM-Polymerschmelze über einen Adapter einem Werkzeug mit konisch ausgeführten multiplen quaderförmigen bzw. konischen zylinderförmigen Öffnungen zugeführt und die sehr dünnflüssige PCM-Polymerschmelze durch die quaderförmigen bzw. zylinderförmigen Öffnungen auf ein grobmaschiges, 15 cm breites Polyamidnetz gegossen, welches sich direkt unterhalb des multiplen Gießwerkzeuges befand. Nach Erkalten der PCM-Polymerschmelzeangüsse wurde das Angusswerkzeug angehoben und das Polyamidgewebe jeweils auf einem Förderband weiterbewegt und anschließend ein erneuter Anguss über das Schmelzewerkzeug durchgeführt.

Auf diese Art und Weise wurde ein Polyamidnetzband mit darauf eng applizierten PCM-Polymerquadern bzw. PCM-PolymerZylindern erlangt. Die Mindestabstände zwischen den PCM-Polymerquadern bzw. PCM-Polymerzylindern betrugen 1 mm. Die Höhe der PCM-Polymerquader bzw. der PCM-Polymerzylinder betrug 3 mm. Die PCM-Polymer-Quader wiesen eine Kantenlänge von 10 mm auf, und die PCM-Polymer-Zylinder hatten einen Durchmesser von 10 mm. Das Flächengewicht des erlangten Polyamidgewebes mit den aufgebrachten PCM-Polymerquadern bzw. PCM-Polymerzylinder betrug entsprechend 1950 und 1850 g/m$^2$. Die Wärmespeicherkapazität der erlangten Polyamidgewebe-PCM-Polymer-Konstrukte lag bei 200 J/g bzw. flächenbezogen bei 390 KJ/m$^2$ und 370 KJ/m$^2$.

Die quaderförmigen bzw. zylinderförmigen PCM-Polymer-Angüsse waren sehr fest mit dem Polyamidgewebe-Gewebe verbunden, auch bei der entsprechenden Phasenwechseltemperatur. Die so erlangten Matten aus Polyamidgewebe und darauf appliziertem PCM-Polymerangüssen (siehe Bilder 1 und 2) konnten als Zylinder aufgewickelt werden bzw. als Mehrfachlagen übereinander gelegt werden, wobei Beladungstests mit warmer Luft bzw. Wasser zeigten, dass diese Mattenkonstruktionen den ein- und austretenden Wärmeströmungen nur einen sehr geringen Strömungswiderstand entgegensetzten.

Ein besonderer Vorteil der Erfindung besteht darin, dass man diese PCM-Flächengebilde auch im kalten Zustand jederzeit verformen kann zu Rollen- und Stapelartikeln und das auch, wenn PCM-Angussstücke mit hoher Phasenübergangstemperatur verwendet werden. Die Steifigkeit der relativ harten PCM-Angussstücke stört hier nicht, da die PCM-Flächengebilde an den Kantenzwischenabständen sehr flexibel verformbar sind.

In einer Testkammer wurden mehrere Stücke dieser Polyamidgewebe-PCM-Polymer-Matten in zwei seitlichen Rahmen fest eingespannt und so kurz hintereinander angeordnet. In die Testkammer wurde warme Luft von 40 °C eingeleitet, um die Polyamidgewebe-PCM-Polymer-Matten mit Wärme zu beladen, und das darin enthaltene PCM (Nacolether 12) bei 32 °C aufzuschmelzen. Bei dieser Phasenübergangstemperatur blieben die Polyamidgewebe-PCM-Polymer-Matten fest eingespannt und hingen nicht durch und flatterten auch nicht, wie man dies sonst bei dickeren PCM-Polymerfolien bzw. PCM-Polymerplatten beobachtet.

**Beispiel 2:**

Ähnlich wie in Beispiel 1 angeführt, wurden wieder PCM-Polymergranulate der Zusammensetzung:

- 80 Gew.% PCM-Material (Nacol®ether 12 der Sasol GmbH , langkettiger Dialkylether)
- 10 Gew.% Styrol-Block-Copolymer SEBS (Polystyrol-b-poly(ethylen/butylen)-b-polystryol; Septon 8004 der Kuraray Co. Ltd.)
- 5 Gew.% OH-terminiertes Styrol-Block-Copolymer (Septon HG 252 der Kuraray Co. Ltd.)
- 5 Gew.% kristallines Ethylen-Butylen Copolymer (Typ 6201 B der JSR Dynaron)

im Doppelschneckenextruder ZSE 40 Leistritz und nachfolgenden Unterwassergranulator hergestellt, getrocknet und in einem Einschneckenextruder mittels speziellen Angusswerkzeugs auf ein grobmaschiges Polyamidgewebe appliziert. Das Polyamidgewebe mit den PCM-Polymer-Angüssen (quaderförmig bzw. zylinderförmig) wurde dann beidseitig mit einer 5 Masse-%igen - PA-Lösung (aus Ultramid 1C) besprüht und das überschüssige Lösungsmittel bei Raumtemperatur verdampft.

Die PA-Beschichtung wies eine Dicke von ca. 5 μm auf. Sie erwies sich als eine 100 %-ige Barriere gegen das Ausschwitzen von PCM.

**Beispiel 3:**

Ähnlich wie in Beispiel 1 angeführt, wurden wieder PCM-Polymergranulate im Doppelschneckenextruder ZSE 40 Leistritz und nachfolgenden Unterwassergranulator hergestellt, getrocknet und in einem Einschneckenextruder mit einem speziellen Angusswerkzeug nunmehr auf ein grobmaschiges Polyamidgewebe appliziert.
Die stoffliche Zusammensetzung des PCM-Polymers wurde hier aber geändert:

- 70 Gew.% PCM-Material (Nacol®ether 16 der Sasol GmbH , langkettiger Dialkylether)
- 8 Gew.% Styrol-Block-Copolymer SEEPS (Septon 4055 der Kuraray Co. Ltd)
- 4 Gew.% OH-terminiertes Styrol-Block-Copolymer (Septon HG 252 der Kuraray Co. Ltd)
- 3 Gew.% kristallines Ethylen-Butylen Copolymer (Typ 6201 B der JSR Dynaron)
- 15 Gew.% Zinkoxid-Pulver
  im Doppelschneckenextruder ZSE 40 Leistritz und nachfolgenden Unterwassergranulator hergestellt, getrocknet und in einem Einschneckenextruder mittels speziellen Angusswerkzeugs auf ein grobmaschiges Polyamidgewebe appliziert.

Das Zinkoxid verbesserte nicht nur die Wärmeleitfähigkeit von 0,2 W/mK (reiner PCM-Polymer-Compound) auf den Wert 0,6 W/mK, sondern bewirkte andererseits auch eine starke Absenkung des Ausschwitzens von Paraffin bei Phasenübergang des PCM.
Die erlangten Polyamidgewebe-PCM-Polymermatten wurden durch Konfektionierung mit einem zusätzlichen dünnen Baumwollüberzug versehen, um diversen Anwendungsaspekten Rechnung zu tragen.
Die Wärmespeicherkapazität der erlangten PCM-Polymer-Granulate betrug 175 J/g und die Wärmekapazität der erlangten Polyamidgewebe-PCM-Polymer-Flächengebilde betrug 160 J/g. Flächenbezogen waren dies 341 KJ/m$^2$ bei Angüssen mit einer PCM-Polymerquaderstruktur bzw. 323 KJ/m$^2$ bei PCM-Polymerangüssen mit einer Zylinderstruktur.

**Beispiel 4**

Bei vorgegebenen Formen und Maßen der PCM-Polymerangussstücke und Wärmespeicherkapazitäten des PCM kann der Besatz des Trägermaterials mit den PCM-Polymerangussstücken berechnet werden und tabellarisch erfasst werden. Mit Hilfe solcher Tabellen gelingt es, die für eine Anwendung benötigte Dimensionierung zu kalkulieren.
Den Berechnungen in diesem Beispiel wurde ein synthetisches Paraffin mit einer Phasenwechselenthalpie von 248 kJ/kg*15 K zugrunde gelegt. Durch die Compoundierung reduziert sich diese Kapazität um 20%. Eine vollflächige Platte einer Höhe von 5 mm basierend auf dieser Rezeptur hätte eine Kapazität von 794 kJ/m$^2$. Dieser Wert wurde als Basisvergleichswert zur Berechnung des verbleibenden Anteils herangezogen.
Wird z. B. eine Kapazität von 600 kJ/m$^2$ benötigt (Vorgaben: Höhe: 5 mm, oben beschriebenes Compound) wird aus Tabelle 1 ersichtlich:

1. Runde Grundform scheidet aus
2. Quadratische Grundform bietet die geforderte Kapazität mit einem Abstand zwischen 1-2 mm.
3. Berechnung:

```
600 (kJ/m²)/198 kJ/kg*15K=3,03 kg PCM (obiger Spezifikation)


    3,03 kg PCM / 0,8 (g/cm³) = 3,79 dm³ = 3.790 cm³


      3.790 cm³ / 0,5 cm³ = 7.580 Stück
```

√7580 = 87, abgerundet auf 87

$$100 \ (cm)/87 = 1,15 \ mm$$

Der Abstand zwischen den PCM-Angussstücken muss bei einer Dicke von 5 mm und Kantenlänge von 1 cm zum Erreichen einer Kapazität von 600 kJ/m$^2$ 1,15 mm betragen

Tabelle 1: Vergleich der Wärmespeicherkapazität von mit separaten PCM-Polymerangussstücken besetzten Trägerstrukturen zu PCM-Flächengebilden mit durchgängiger PCM-Beschichtung Höhe der Angussstücke = 5 mm

| Abstand zwischen den PCM-Teilchen in mm | Kapazität/m$^2$ in J | Form Grundfläche Angussstücke | Kapazität in % gegenüber der vollflächi gen Bedeckung (5 mm) |
|---|---|---|---|
| 0,5 | 716 | quadratisch | 90 |
| 1 | 643 | quadratisch | 81 |
| 2 | 547 | quadratisch | 69 |
| 3 | 458 | quadratisch | 58 |
| 0,5 | 562 | rund | 71 |
| 1 | 505 | rund | 64 |
| 2 | 429 | rund | 54 |
| 3 | 360 | rund | 45 |

Wie aus der Tabelle zu sehen ist, kann mit quadratischen Grundflächen eine höhere Flächenkapazität erzielt werden. Runde Grundflächen erbringen geringere Flächenkapazitäten. Bei Versatz zueinander werden diese Werte zwar etwas besser, erreichen aber nicht die der quadratischen.
Die Spalte "Kapazität in % gegenüber der vollflächigen Bedeckung" zeigt an, wieviel Kapazität bei unterschiedlichen Geometrien (quadratisch bzw. rund) mit welchem Abstandsmaß gegenüber einer vollflächigen PCM-Schicht übrig bleibt.
Annahme der Höhe der Angussstücke bzw. Höhe der PCM-Schicht ist bei 5 mm, bei Reduzierung der Höhe nimmt die Kapazität linear ab und ist daher nicht in der Tabelle erfasst.
Bei den Beladungsangaben in % kommt es zu leichten Sprüngen. Diese resultieren aus der Tatsache, dass je nach Teilchenabstand am Rand nicht mehr ganze Reihen zustande kommen. In der Praxis könnten die mechanisch geschnitten werden, dann würden die Verhältnisse sich linear verhalten. Alle anderen Grundgeometrien (z. B. Dreieck, Vielecke etc.) liegen mit der Flächenbeladungsausnutzung jeweils zwischen den Werten von Quadrat und Kreis und werden daher in der Tabelle nicht weiter aufgeführt.

**Patentansprüche**

1. Flexibles PCM-Flächengebilde mit hoher latenter Wärmeenergie-Speicherdichte, **gekennzeichnet durch** eine flexiblen 2-dimensionale Trägerstruktur mit darauf an der Oberfläche applizierten und mit der Trägerstruktur fest verbundenen, separat in einem Abstand von mindestens 0,5 mm angeordneten geometrisch definierten Strukturen eines polymergebundenen Phasenwechselmaterials, wobei das Phasenwechselmaterial durch mindestens zwei Polymere gebunden ist, von denen mindestens ein Polymer ausgewählt ist aus der Gruppe der styrolhaltigen Blockcopolymere und mindestens ein Polymer ausgewählt ist aus der Gruppe der styrolfreien Ethylen/Butylen-Copolymere, wobei das Flächengebilde auch bei Phasenwechsel formstabil ist, eine latente Wärmeenergie-Speicherdichte von 100 bis 250 J/g bzw. 300 bis 1000 kJ/m$^2$ aufweist und gerollt, gefaltet, gewickelt, zugeschnitten oder mehrlagig konfektioniert werden kann.

2. Flexibles PCM-Flächengebilde nach Anspruch 1, **dadurch gekennzeichnet, dass** die Trägerstruktur Gewebe, Vliesstoffe, Gewirke, Gestricke, Geflechte aus Fasern oder Garnen oder Foliebändchen, Folien oder Membranen umfasst, vorzugsweise Gewebe mit einer großen Maschenweite oder poröse oder geflochtene Strukturen umfasst.

3. Flexibles PCM-Flächengebilde nach Anspruch 2, **dadurch gekennzeichnet, dass** das Material der Trägerstruktur Polyamid, Polyester, Polypropylen, Cellulose, Kohlenstoff, Metall, Glas, Naturfasern oder Mischungen aus diesen Materialien umfasst.

**4.** Flexibles PCM-Flächengebilde nach einem oder mehreren der Ansprüche 1 bis 3, **dadurch gekennzeichnet, dass** es 1 bis 10 Gew.-% Trägerstruktur und 90 bis 99 Gew.-% darauf applizierten geometrisch definierten Strukturen des polymergebundenen Phasenwechselmaterials umfasst.

**5.** Flexibles PCM-Flächengebilde nach einem oder mehreren der Ansprüche 1 bis 4, **dadurch gekennzeichnet, dass** das polymergebundene Phasenwechselmaterial 10 bis 30 Gew.-% Trägerpolymere und 90 bis 70 Gew.-% Phasenwechselmaterial umfasst.

**6.** Flexibles PCM-Flächengebilde nach einem oder mehreren der Ansprüche 1 bis 5, **dadurch gekennzeichnet, dass** es 5 bis 20 Gew.-% an anorganischen oder organischen Additiven enthält, bezogen auf das Gewicht des gesamten Angussmaterials.

**7.** Flexibles PCM-Flächengebilde nach einem oder mehreren der Ansprüche 1 bis 6, **dadurch gekennzeichnet, dass** es oberflächlich mit einer 3 bis 10 $\mu$m dicken Schicht aus Polymer-, Metall- oder Keramikmaterial oder mit einem textilen Flächenüberzug aus diesen Materialien beschichtet ist.

**8.** Flexibles PCM-Flächengebilde nach einem oder mehreren der Ansprüche 1 bis 7, **dadurch gekennzeichnet, dass** das Flächengewicht des polymergebundenen Phasenwechselmaterials 1 bis 4 kg/m$^2$ beträgt.

**9.** Flexibles PCM-Flächengebilde nach einem oder mehreren der Ansprüche 1 bis 8, **dadurch gekennzeichnet, dass** es von flüssigen oder gasförmigen Wärmemedien durchströmbar ist.

**10.** Verfahren zur Herstellung von flexiblen PCM-Flächengebilden mit hoher latenter Wärmeenergie-Speicherdichte nach einem oder mehreren der Ansprüche 1 bis 9, **dadurch gekennzeichnet, dass** eine plastifizierte Masse eines polymergebundenen Phasenwechselmaterials in geometrisch definierten Strukturen auf einem gewebeartigen Trägermaterial fest fixiert wird, indem das schmelzverflüssigte polymergebundene Phasenwechselmaterial auf das Trägermaterial als sphärische, quadratische, rechtwinklige oder polygonale separate Formkörper mit einer Dicke von 1 bis 10 mm, bevorzugt 1 bis 5 mm, durch Spritzguss, Aufspritzen, Aufstreichen oder druckloses Vergießen mittels einer Formvorrichtung in einem kontinuierlichen oder diskontinuierlichen Verfahren aufgebracht wird.

**11.** Verwendung des flexiblen PCM-Flächengebildes nach einem oder mehreren der Ansprüche 1 bis 9 zur Speicherung von Wärme und Kälte und zur Wärmeregulierung, insbesondere im Gebäudemanagement, Heat-sink-Applikationen in der Elektrotechnik, Wärme / Kältemanagement im Fahrzeugbau oder zur ein- oder mehrlagigen Umhüllung von Rohrleitungen.

**Claims**

**1.** Flexible PCM sheet material with high latent thermal energy storage density, **characterized by** a flexible 2-dimensional carrier structure having geometrically defined structures of a polymer-bound phase change material arranged separately at a spacing of at least 0.5 mm, applied on the surface thereof and connected firmly to the carrier structure, the phase change material being bound by at least two polymers, of which at least one polymer is selected from the group of styrene-containing block copolymers and at least one polymer is selected from the group of styrene-free ethylene/butylene copolymers, the sheet material being dimensionally stable even on phase change, having a latent thermal energy storage density of 100 to 250 J/g and/or 300 to 1000 kJ/m$^2$, and being convertible in rolled, folded, wound, cut-to-size or multi-ply form.

**2.** Flexible PCM sheet material according to Claim 1, **characterized in that** the carrier structure comprises wovens, nonwovens, loop-formed knits, loop-drawn knits, braids of fibers or yarns or film ribbons, films or membranes, and preferably comprises wovens having a large mesh size or porous or braided structures.

**3.** Flexible PCM sheet material according to Claim 2, **characterized in that** the material of the carrier structure comprises polyamide, polyester, polypropylene, cellulose, carbon, metal, glass, natural fibers or mixtures of these materials.

**4.** Flexible PCM sheet material according to one or more of Claims 1 to 3, **characterized in that** it comprises 1 to 10 % by weight of carrier structure and 90 to 99 % by weight of geometrically defined structures of the polymer-bound phase change material applied to said carrier structure.

**5.** Flexible PCM sheet material according to one or more of Claims 1 to 4, **characterized in that** the polymer-bound phase change material comprises 10 to 30 % by weight of carrier polymers and 90 to 70 % by weight of phase change material.

**6.** Flexible PCM sheet material according to one or more of Claims 1 to 5, **characterized in that** it contains 5 to 20 % by weight of inorganic or organic additives, based on the weight of the total cast-on material.

**7.** Flexible PCM sheet material according to one or more of Claims 1 to 6, **characterized in that** it is coated superficially with a layer 3 to 10 $\mu$m thick of polymeric, metallic or ceramic material or with a textile sheet covering comprising these materials.

**8.** Flexible PCM sheet material according to one or more of Claims 1 to 7, **characterized in that** the areal weight of the polymer-bound phase change material is 1 to 4 kg/m$^2$.

**9.** Flexible PCM sheet material according to one or more of Claims 1 to 8, **characterized in that** it is flow-traversable by liquid or gaseous thermal media.

**10.** Method for producing flexible PCM sheet materials with high latent thermal energy storage density according to one or more of Claims 1 to 9, **characterized in that** a plastified mass of a polymer-bound phase change material is fixed firmly in geometrically defined structures on a fabriclike carrier material, the melt-liquefied, polymer-bound phase change material being applied to the carrier material as spherical, square, rectangular or polygonal separate shaped elements having a thickness of 1 to 10 mm, preferably 1 to 5 mm, by injection molding, spraying, spreading or unpressurized casting by means of a forming apparatus in a continuous or discontinuous process.

**11.** Use of the flexible PCM sheet material according to one or more of Claims 1 to 9 for storing heat and cold and for thermal regulation, especially in building management, heat sink applications in electrical engineering, heat/cold management in vehicle construction, or for single-ply or multi-ply jacketing of pipelines.

**Revendications**

**1.** Structure plane flexible en matériau à changement de phase avec densité d'accumulation d'énergie thermique latente élevée, **caractérisée par** une structure de support bidimensionnelle flexible avec des structures d'un matériau à changement de phase lié à des polymères définies de manière géométrique agencées séparément à une distance d'au moins 0,5 mm, reliées fermement à la structure de support et appliquées dessus au niveau de la surface, dans laquelle le matériau à changement de phase est lié par au moins deux polymères, dont au moins un polymère est choisi dans le groupe des copolymères blocs contenant du styrène et au moins un polymère est choisi dans le groupe des copolymères éthylène/butylène sans styrène, dans laquelle la structure plane est également indéformable en cas de changement de phase, présente une densité d'accumulation d'énergie thermique latente de 100 à 250 J/g ou de 300 à 1000 kJ/m$^2$ et peut être enroulée, pliée, bobinée, découpée ou confectionnée en plusieurs couches.

**2.** Structure plane flexible en matériau à changement de phase selon la revendication 1, **caractérisée en ce que** la structure de support comprend des tissus, des non-tissés, des tissés, des tricots, des tresses en fibres ou fils ou bandelettes de film, films ou membranes, de préférence des tissus à grand maillage ou des structures poreuses ou tressées.

**3.** Structure plane flexible en matériau à changement de phase selon la revendication 2, **caractérisée en ce que** le matériau de la structure de support comprend du polyamide, du polyester, du polypropylène, de la cellulose, du carbone, du métal, du verre, des fibres naturelles ou des mélanges de ces matériaux.

**4.** Structure plane flexible en matériau à changement de phase selon une ou plusieurs des revendications 1 à 3, **caractérisée en ce qu'**elle comprend 1 à 10 % en poids de structure de support et 90 à 99 % en poids de structures du matériau à changement de phase lié à des polymères définies de manière géométrique appliquées dessus.

**5.** Structure plane flexible en matériau à changement de phase selon une ou plusieurs des revendications 1 à 4, **caractérisée en ce que** le matériau à changement de phase lié à des polymères comprend 10 à 30% en poids de polymères support et 90 à 70 % en poids de matériau à changement de phase.

**6.** Structure plane flexible en matériau à changement de phase selon une ou plusieurs des revendications 1 à 5, **caractérisée en ce qu'**elle contient 5 à 20 % en poids d'additifs anorganiques ou organiques, par rapport au poids de l'ensemble de matériau injecté,

**7.** Structure plane flexible en matériau à changement de phase selon une ou plusieurs des revendications 1 à 6, **caractérisée en ce qu'**elle est revêtue en surface d'une couche de 3 à 10 $\mu$m d'épaisseur de matériau polymère, métallique ou céramique ou d'un revêtement plan textile formé à partir de ces matériaux.

**8.** Structure plane flexible en matériau à changement de phase selon une ou plusieurs des revendications 1 à 7, **caractérisée en ce que** le poids par unité de surface du matériau à changement de phase lié à des polymères est de 1 à 4 kg/m$^2$.

**9.** Structure plane flexible en matériau à changement de phase selon une ou plusieurs des revendications 1 à 8, **caractérisée en ce qu'**elle peut être traversée par des milieux de chauffage liquides ou gazeux.

**10.** Procédé de fabrication de structures planes flexibles en matériau à changement de phase avec densité d'accumulation d'énergie thermique latente élevée selon une ou plusieurs des revendications 1 à 9, **caractérisé en ce qu'**une masse plastifiée d'un matériau à changement de phase lié à des polymères est fixé fermement dans des structures définies de manière géométrique sur un matériau de support de type tissu, par le fait que le matériau à changement de phase lié à des polymères fluidifié par fusion est appliqué sur le matériau de support en tant que corps moulé séparé sphérique, carré, rectangulaire ou polygonal avec une épaisseur de 1 à 10 mm, de préférence de 1 à 5 mm, par moulage par injection, surmoulage, étalage ou versage sans pression au moyen d'un dispositif de moulage dans une procédé continu ou discontinu.

**11.** Utilisation de la structure plane flexible en matériau à changement de phase selon une ou plusieurs des revendications 1 à 9 pour l'accumulation de chaleur et de froid et pour la régulation thermique, en particulier dans la gestion des bâtiments, les applications heat-sink dans l'électrotechnique, la gestion chaud / froid dans la construction de véhicules ou pour l'enveloppage à une ou plusieurs couches de conduites.

Bild 1

Bild 2

Bild 3

Bild 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20020164474 A
- GB 2495938 A
- US 2002164474 A
- DE 10022287
- US 20120064327 A1